# EUROPÄISCHE PATENTSCHRIFT

(11) **EP 2 138 298 B1**
(45) Veröffentlichungstag und Bekanntmachung des Hinweises auf die Patenterteilung: **03.12.2014**
(21) Anmeldenummer: 09163763.7
(22) Anmeldetag: 25.06.2009
(51) Int. Cl.: B29C 49/42, A61L 2/08, B65B 55/08, B67C 3/26, B29C 49/64

(54) **Vorrichtung und Verfahren zum Herstellen von Kunststoffbehältnissen**
Device and method for manufacturing plastic containers
Dispositif et procédé de fabrication de récipients en plastique

(30) Priorität: 27.06.2008 DE 102008030156
(43) Veröffentlichungstag der Anmeldung: 30.12.2009
(60) Teilanmeldung: 14166851.7
(73) Patentinhaber: Krones AG, 93073 Neutraubling (DE)
(72) Erfinder: Krüger, Jochen, 93095 Hagelstadt (DE); Faltermeier, Manfred, 84094, Elsendorf (DE)
(74) Vertreter: Bittner, Bernhard

(56) Entgegenhaltungen:
- EP-A1- 2 420 258
- WO-A1-2008/055685
- WO-A2-2008/125216
- WO-A2-2010/055113
- WO-A2-2011/029856

## Beschreibung

Die Erfindung bezieht sich auf eine Vorrichtung und ein Verfahren zum Herstellen von Kunststoffbehältnissen und insbesondere von Kunststoffbehältnissen für Getränke. Es wird jedoch auch darauf hingewiesen, dass die hergestellten Behältnisse auch für andere Flüssigkeiten verwendbar sind. Aus dem Stand der Technik ist eine Vielzahl von Vorrichtungen zum Herstellen von Kunststoffbehältnissen bekannt. Dabei ist es insbesondere bekannt, dass sogenannte Kunststoffvorformlinge einer Blaseinrichtung zugeführt werden wobei diese Blaseinrichtung mit Hilfe von Luftdruck die Kunststoffvorformlinge zu Kunststoffbehältnissen expandiert.

Im Stand der Technik tritt jedoch dabei des Öfteren das Problem auf, dass während des Expansionsvorgangs oder auch danach Verunreinigungen der Behältnisse auftreten können oder aber bereits die Vorformlinge verunreinigt sind. Aus diesem Grunde ist es im Stand der Technik bekannt, die erzeugten Behältnisse mit Hilfe von Chemikalien zu reinigen.

So ist beispielsweise aus der EP 0996530 eine chemische Preformentkeimung vor dem Einlauf in einen Heizofen bekannt oder auch das Injizieren von wärmeaktivierbarem Desinfektionsmittel in die Preform und ein anschließendes Erwärmen der Preform in einer Blasmaschine auf eine Aktivierungstemperatur des Desinfektionsmittels sowie eine nachfolgende Erwärmung auf eine Verformungstemperatur. Weiterhin offenbart die US 6,692,684 ein Verfahren, bei dem die Preforms zunächst über den Taupunkt des Desinfektionsmittels erwärmt werden und das Desinfektionsmittel gasförmig eingebracht wird und anschließend eine Erwärmung auf Umformungstemperatur erfolgt.

Aus der WO 20081055685 A1 ist eine Vorrichtung zur Herstellung von Kunststoffbehältnissen bekannt. Dabei können je nach herzustellender Flaschenart alternative Behandlungsmethoden angewandt werden.

Die nachveröffentlichte Druckschrift WO 2008/125216 A2 beschreibt eine Behälterherstellungsvorrichtung und ein Herstellverfahren für Formkörper. Dabei werden im Rahmen einer Blasformmaschine sowohl Maschinenteile als auch Kunststoffvorformlinge sterilisiert.

Die Verwendung derartiger Chemikalien ist jedoch sowohl aus umweltpolitischen Gründen als auch aus Kostengründen problematisch. Der vorliegenden Erfindung liegt daher die Aufgabe zugrunde, eine Vorrichtung und ein Verfahren zur Herstellung von Kunststoffbehältnissen zur Verfügung zu stellen, welches effizient arbeitet und gleichzeitig den Einsatz von Chemikalien zumindest reduziert.

Dies wird erfindungsgemäß durch eine Vorrichtung zum Herstellen von Kunststoffbehältnissen nach Anspruch 1 und ein Verfahren zum Herstellen von Kunststoffbehältnissen nach Anspruch 9 erreicht. Vorteilhafte Ausführungsformen und Weiterbildungen sind Gegenstand der abhängigen Ansprüche.

Im Folgenden bedeutet "erste Sterilisationseinrichtung", dass es sich um eine "Strahlungseinrichtung zur Erzeugung von Elektronenstrahlung" handelt.

Des Weiteren bedeutet "zweite Sterilisationseinrichtung", dass es sich um eine "Strahlungseinrichtung zur Erzeugung von UV-Strahlung" handelt.

Eine erfindungsgemäße Vorrichtung zum Herstellen von Kunststoffbehältnissen weist eine Umformungseinrichtung auf, welche Kunststoffvorformlinge zu Kunststoffbehältnissen umformt sowie eine erste Transporteinrichtung, welche die Kunststoffvorformlinge der Umformungseinrichtung zuführt und eine zweite Transporteinrichtung, welche die Kunststoffbehältnisse von der Umformungseinrichtung abführt. Erfindungsgemäß weist die Vorrichtung eine erste Sterilisationseinrichtung zum Sterilisieren der Kunststoffvorformlinge auf, welche in einer Transportrichtung der Kunststoffvorformlinge vor der Umformungseinrichtung angeordnet ist sowie eine zweite Sterilisationseinrichtung zum Sterilisieren der Kunststoffbehältnisse, welche in einer Transportrichtung der Kunststoffbehältnisse nach der Umformungseinrichtung angeordnet ist.

Erfindungsgemäß weist wenigstens eine Sterilisationseinrichtung wenigstens eine Strahlungsquelle auf, deren Bewegung an die Bewegung der Kunststoffvorformlinge oder die Bewegung der Kunststoffbehältnisse gekoppelt ist.

Erfindungsgemäß weist die erste Sterilisationseinrichtung eine Strahlungsquelle auf, mittels derer die Kunststoffvorformlinge mit Elektronenstrahlen beaufschlagt werden. Damit kann bevorzugt im Rahmen der Sterilisation durch die erste Sterilisationseinrichtung auf den Einsatz von Chemikalien verzichtet werden.

Erfindungsgemäß weist die zweite Sterilisationseinrichtung eine Strahlungsquelle auf, mittels derer die Kunststoffbehältnisse mit einer Strahlung beaufschlagt werden. Erfindungsgemäß beaufschlagt die erste Sterilisationseinrichtung die Kunststoffvorformlinge mit Elektronenstrahlen wohingegen die zweite Sterilisationseinrichtung die Kunststoffbehältnisse mit UV-Strahlung beaufschlagt. Damit findet durch die zweite Sterilisationseinrichtung eine Nachsterilisation der bereits durch die erste Sterilisationseinrichtung vorsterilisierten Behältnisse statt. Bevorzugt dient die zweite Sterilisationseinrichtung insbesondere dazu, um nach der ersten Sterilisation aufgetretene Verkeimungen zu beseitigen.

Durch den Blasprozess und den weiteren Transport verursachte Verkeimungen werden dadurch beseitigt, dass, bevorzugt in einer Transfertransporteinrichtung zwischen der Umformungseinheit und beispielsweise einer Befüllungseinrichtung, eine weitere Sterilisationseinrichtung vorgesehen ist, die ebenso bevorzugt keine chemischen Mittel einsetzt. Dazu werden die Kunststoffbehältnisse bevorzugt mittels eines Lineartransfers mit UV - Lampen bestrahlt. Dabei ist es möglich, dass die Kunststoffbehältnisse in ihren Halteeinrichtungen beispielsweise sog. Neckhandlingklammern gedreht werden, damit durch das Neckhandling keine Schatten entstehen, die eine sichere Entkeimung verhindern.

Es wird darauf hingewiesen, dass es auch möglich ist, die erste Sterilisationseinrichtung in einem Bereich der Umformungseinrichtung und insbesondere in einem Einlaufbereich derselben anzuordnen. Auch wäre es denkbar, dass die Sterilisation zumindest zeitweise zeitgleich mit dem Expansionsvorgang stattfindet. Daneben könnte auch die zweite Sterilisationseinrichtung in einem Bereich der Umformungseinrichtung und/oder auch in einem Bereich eines Auslaufs derselben vorgesehen sein.

Auch wäre es möglich, dass in den einzelnen Blasstationen der Umformungseinrichtung Sterilisationseinrichtungen angeordnet sind. So könnten beispielsweise an den Innenwandungen von Blasformen, welche den zu expandierenden Behältnissen zugewandt sind, Strahlungsquellen wie UV-Lampen, angeordnet sein, welche die Behältnisse während des Expansionsvorgangs bestrahlen. Auch wäre es möglich, insbesondere UV - Strahlungsquellen derart anzuordnen, dass die Kunststoffvorformlinge während des Schließens einer Blasform oder die Kunststoffbehältnisse während des Öffnens einer Blasform mit UV - Strahlung beaufschlagt werden.

Daneben wäre es auch möglich, während des Expansionsvorgangs, beispielsweise über eine Reckstange, ein Sterilisationsmedium, wie beispielsweise Wasserstoffperoxid in die Behältnisse einzuführen

Die oben beschriebene Sterilisation der Vorformlinge bietet den Vorteil, dass die Oberflächen dieser Vorformlinge kleiner sind als diejenige der bereits fertig geblasenen Behältnisse und daher der Aufwand, insbesondere beim Einsatz von Elektronenstrahlen geringer ist.

Bei einer weiteren bevorzugten Ausführungsform weist die Vorrichtung eine Dreheinrichtung auf, welche die Vorformlinge während der Sterilisation durch die erste und/oder die zweite Sterilisationseinrichtung dreht. Damit ist es möglich, dass eine Strahlungsquelle drehfest angeordnet ist und die Vorformlinge diese gegenüber gedreht werden, um einen größeren Bereich der Vorformlinge zu sterilisieren. Dabei führt die erste Sterilisationseinrichtung insbesondere auch einen Sterilisation der Innenwandung der Vorformlinge durch. Es wäre jedoch auch möglich, dass vor der Umformungseinrichtung mehrere Sterilisationseinrichtungen vorgesehen sind, welche die Behältnisse sowohl an ihrer Außenwandung als auch an ihrer Innenwandung und insbesondere mit Elektronenstrahlen beaufschlagen.

Bevorzugt ist eine Vielzahl derartiger Dreheinrichtungen vorgesehen, wobei bevorzugt die Dreheinrichtungen an Transportelementen, wie beispielsweise Greifklammern vorgesehen sind, so dass mehrere Vorformlinge gleichzeitig transportiert und gedreht werden können.

Bei einer weiteren vorteilhaften Ausführungsform weist wenigstens eine Sterilisationseinrichtung und insbesondere die erste Sterilisationseinrichtung wenigstens eine Strahlungsquelle auf, deren Bewegung an die Bewegung der Kunststoffvorformlinge oder die Bewegung der Kunststoffbehältnisse gekoppelt ist.

Dies bedeutet, dass diese Strahlungsquelle mit den Kunststoffbehältnissen mitgeführt wird. Vorzugsweise findet diese Koppelung bei der ersten Sterilisationsvorrichtung statt. Es wäre jedoch umgekehrt auch möglich, dass die Behältnisse nicht gedreht werden und die besagte Strahlungsquelle der Sterilisationseinrichtung gedreht wird. Auch ist es möglich, dass die Strahlungsquellen stationär angeordnet sind und die Kunststoffvorformlinge sich gegenüber diesen stationär angeordneten Strahlungsquellen bewegen um auf diese Weise ebenfalls eine möglichst umfangreiche Sterilisation der Kunststoffvorformlinge zu erreichen. Weiterhin wäre es auch möglich, dass die Strahlungsquelle in einer Längsrichtung der Kunststoffvorformlinge diesen gegenüber bewegbar ist.

Dabei werden die Kunststoffvorformlinge mit Elektronenstrahlung (E-Beam) entkeimt. Dabei ist es möglich, insbesondere mit geringer Dosis von einer Mündung der Behältnisse her zu entkeimen, es wäre jedoch auch möglich, durch eine Seitenwand der Vorformlinge hindurch zu entkeimen, wobei hierzu bevorzugt hohe Beschleunigungsspannungen verwendet werden, um eine Durchdringung des Materials zu erhalten. Dabei kann, wie erwähnt, die Bestrahlung nach der Sortierung, im Einlaufrad der Heizeinrichtung, in der Heizeinrichtung oder im Transfer zwischen der Heizeinrichtung und der Umformungseinrichtung oder auch in einem Auslaufstern der Umformungseinrichtung bzw. Blasmaschine, oder auch an mehreren oder allen dieser Positionen erfolgen.

Bei einer weiteren vorteilhaften Ausführungsform ist wenigstens ein Abschnitt wenigstens einer Strahlungsquelle in die Mündung der zu sterilisierenden Kunststoffvorformlinge einführbar. So ist es möglich, dass die Strahlungsquelle ein Austrittsfenster aufweist, durch welches hindurch Elektronen hindurchtreten können. Diese Mündung kann dabei in einem Kopf angeordnet sein, der so dimensioniert ist, dass er durch die Mündung in den Innenraum des Kunststoffvorformlings einführbar ist.

Bei einer weiteren vorteilhaften Ausführungsform weist die Vorrichtung eine Heizeinrichtung auf, welche die Kunststoffvorformlinge erwärmt. Die so erwärmten Kunststoffvorformlinge können anschließend in der Umformungseinrichtung aufgrund der erhöhten Temperatur leichter zu Behältnissen umgeformt werden wobei die Umformungseinrichtung hierzu bevorzugt Düsen verwendet, welche die Kunststoffvorformlinge mit Luft beaufschlagen um diese so zu expandieren.

Vorzugsweise ist die erste Sterilisationseinrichtung in der Transportrichtung der Kunststoffvorformlinge vor der Heizeinrichtung angeordnet. Es wäre jedoch auch möglich, dass die erste Sterilisationseinrichtung innerhalb der Heizeinrichtung angeordnet ist oder unmittelbar nach der Heizeinrichtung und vor der ersten Umformungseinrichtung. Es könnten auch mehrere Sterilisationseinrichtungen vor der Umformungseinrichtung angeordnet sein, beispielsweise eine erste Sterilisationseinrichtung vor der Heizeinrichtung eine weitere in der Heizeinrichtung und eine weitere nach der Heizeinrichtung und vor der Umformungseinrichtung.

Bei einer weiteren vorteilhaften Ausführungsform ist die zweite Sterilisationseinrichtung in der Transportrichtung der Behältnisse unmittelbar nach der Umformungseinrichtung angeordnet. Auf diese Weise ist es möglich, dass die Behältnisse unmittelbar nach deren Erzeugen ein zweites Mal sterilisiert werden. Unter einer unmittelbaren Anordnung wird dabei insbesondere auch verstanden, dass die Behältnisse direkt von der Umformungseinheit über eine Transporteinrichtung, wie einen Transportstern, an die zweite Sterilisationseinrichtung übergeben werden.

Bevorzugt ist die zweite Sterilisationseinrichtung ebenfalls stationär angeordnet und die Behältnisse werden dieser gegenüber transportiert. Bei einer weiteren vorteilhaften Ausführungsform sind die Umformungseinrichtung und die Sterilisationseinrichtung innerhalb eines Gehäuses angeordnet, wobei dieses Gehäuse in seinem Inneren einen Sterilraum ausbildet. Diese bevorzugte Ausführungsform eignet sich insbesondere für die Abfüllung von sensiblen Getränken. Vorzugsweise ist in einem Bereich der ersten Sterilisationseinrichtung innerhalb des besagten Gehäuses ein zweites Gehäuse vorgesehen, wobei innerhalb des zweiten Gehäuses die erste Sterilisationseinrichtung angeordnet ist.

Dabei ist es möglich, dass innerhalb des zweiten Gehäuses ein weiterer Sterilraum ausgebildet ist, der einen noch höheren Reinheitsgrad aufweist als der Sterilraum innerhalb des ersten Gehäuses. Auf diese Weise ist es möglich, nicht das gesamte in üblicher Weise relativ große Gehäuse als hochwirksamen Sterilraum auszubilden, was mit hohen Kosten einhergehen würde, sondern lediglich einen relativ kleinen Bereich, in dem die erste Sterilisationseinrichtung angeordnet ist.

Die vorliegende Erfindung ist weiterhin auf ein Verfahren zum Herstellen von Kunststoffbehältnissen gerichtet, wobei einer Umformungseinrichtung Kunststoffvorformlinge mittels einer ersten Transproteinrichtung zugeführt werden und aus den Künststoffvorformlingen erzeugte Kunststoffbehältnisse mittels einer zweiten Transporteinrichtung von der Umformungseinrichtung abgeführt werden. Erfindungsgemäß werden die Kunststoffvorformlinge auf ihren Transportpfad vor der Umformungseinrichtung und die aus den Kunststoffvorformlingen erzeugten Kunststoffbehältnisse auf Ihren Transportpfad nach der Umformungseinrichtung sterilisiert.

Erfindungsgemäß weist wenigstens eine Sterilisationseinrichtung wenigstens eine Strahlungsquelle auf, deren Bewegung an die Bewegung der Kunststoffvorformlinge oder die Bewegung der Kunststoffbehältnisse gekoppelt wird.

Im Rahmen umfangreicher Messungen hat sich gezeigt, dass es zwar teilweise möglich wäre, lediglich eine Sterilisationseinrichtung vor oder nach der Umformungseinrichtung vorzusehen, dass jedoch eine besonders kostengünstige und effiziente Vorgehensweise darin besteht, zwei Sterilisationseinrichtungen vorzugsehen, wobei eine Sterilisationseinrichtung vor der Umformungseinrichtung und die zweite Sterilisationseinrichtung nach der Umformungseinrichtung angeordnet ist.

Weitere vorteilhafte Ausführungsformen ergeben sich aus den beigefügten Zeichnungen:

Darin zeigen:
- Fig. 1: eine schematische Darstellung einer erfindungsgemäßen Vorrichtung;
- Fig. 2: eine schematische Darstellung eines Bereichs der Vorrichtung aus Fig. 1, in dem die erste Sterilisationseinrichtung angeordnet ist; und
- Fig. 3: eine schematische Darstellung einer Halterung für einen Kunststoffvorformling.

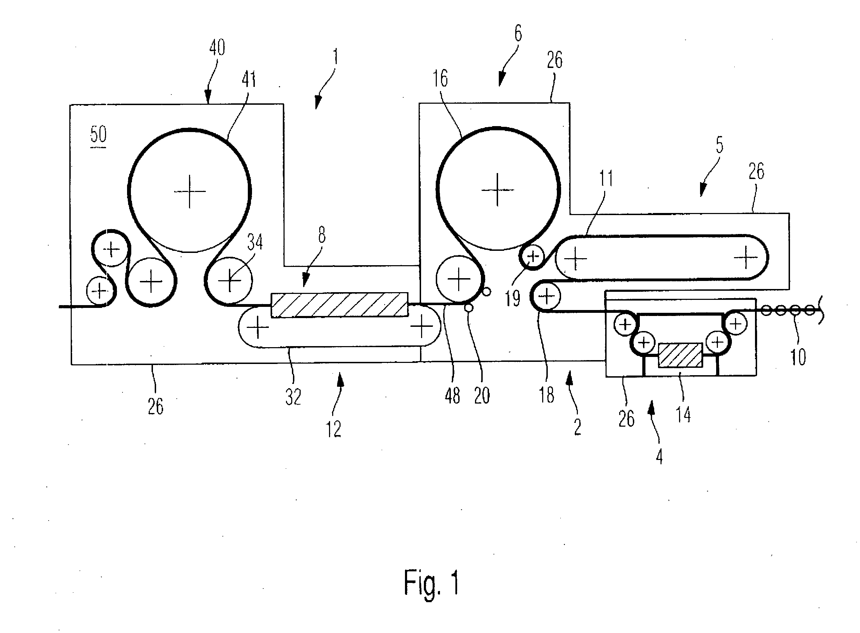

Fig. 1 zeigt eine erfindungsgemäße Vorrichtung 1 zum Herstellen von Behältnissen. Dabei werden Vorformlinge 10 über eine in ihrer Gesamtheit mit 2 bezeichnete Transporteinrichtung zu einer Umformungseinrichtung 6 geführt. Diese Umformungseinrichtung 6 erzeugt aus den Vorformlingen 10 Behältnisse 20, welche anschließend weiter in Richtung einer Befüllungseinrichtung 40 geführt werden. Die Transporteinrichtung 2 setzt sich dabei aus mehreren Transportmitteln zusammen wie beispielsweise einer Transportkette 11, einem Transportstern oder Transportrad 18 und einem weiteren Transportrad 19. Damit werden hier die Vorformlinge 10 bzw. später die Behältnisse 20 im Wesentlichen in Fig. 1 von rechts nach links gefördert.

Die Umformungseinrichtung 6 weist ein Blasrad 16 auf, an dem eine Vielzahl von Blasstationen angeordnet ist, welche die Vorformlinge 10 zu Behältnissen 20 in an sich aus dem Stand der Technik bekannter Weise, expandieren.

Das Bezugszeichen 5 bezieht sich auf eine Heizeinrichtung bzw. einen Ofen innerhalb dessen die Vorformlinge 10 erwärmt werden um anschließend die erwärmten Vorformlinge der Umformungseinrichtung 6 zuzuführen.

Vor dieser Heizeinrichtung 5 ist eine erste Sterilisationseinrichtung 4 angeordnet, welche die Vorformlinge sterilisiert. Dabei weist diese Sterilisationseinrichtung 4 eine Vielzahl von Strahlungsquellen auf (nicht gezeigt), welche die Vorformlinge 10 mittels Elektronenstrahlung sterilisieren. Vorzugsweise sind dabei die einzelnen Strahlungsquellen stationär gehalten und die Behältnisse werden diesen gegenüber bewegt. Die einzelnen Strahlungsquellen sind bevorzugt unabhängig voneinander steuerbar.

Nach Durchlaufen der Umformungseinrichtung 6 gelangen die erzeugten Behältnisse 20 über eine weitere in ihrer Gesamtheit mit 12 bezeichnete zweite Transporteinrichtung in Richtung der Befüllungseinrichtung 40. Dabei kann auch diese zweite Transporteinrichtung 12 eine Vielzahl von Tafeleinheiten aufweisen wie beispielsweise ein Transportrad 48, eine Transportkette 32 und einen Transportstern. Es wäre jedoch auch denkbar, dass die erste Transporteinrichtung 2 und die zweite Transproteinrichtung 12 einteilig, beispielsweise als durchgehend zusammenhängende Transportkette ausgebildet sind.

Das Bezugszeichen 8 bezieht sich auf eine zweite Sterilisationseinrichtung, welche die Behältnisse 20 sterilisiert um beispielsweise neu aufgetretene Verkeimungen zu entfernen. Diese zweite Sterilisationseinrichtung 8 ist dabei bevorzugt unmittelbar nach der Transporteinrichtung 48 angeordnet.

Bei der zweiten Sterilisationseinrichtung 8 handelt es sich um eine mit UV-Strahlung arbeitende Sterilisationseinrichtung. Ausgehend von dieser zweiten Sterilisationseinrichtung 8 gelangen die Behältnisse 20 in Fig. 1 zu der Befüllungseinrichtung und werden damit unmittelbar nach der Sterilisation durch die zweite Sterilisationseinrichtung 8 mit einer Flüssigkeit und insbesondere einem Getränk befüllt. Die Befüllungseinrichtung 40 weist dabei ein Transportrad 41 auf. Anstelle oder neben der Befüllungseinrichtung 40 könnte jedoch auch ein Rinser vorgesehen sein.

Das Bezugszeichen 26 bezieht sich auf ein Gehäuse, welches hier sowohl die Befüllungseinrichtung 40 als auch die beiden Sterilisationseinrichtungen und die Heizeinrichtung sowie die Umformungseinrichtung 6 umgibt. Innerhalb dieses Gehäuses 26 ist eine sterile Umgebung 50 angeordnet.

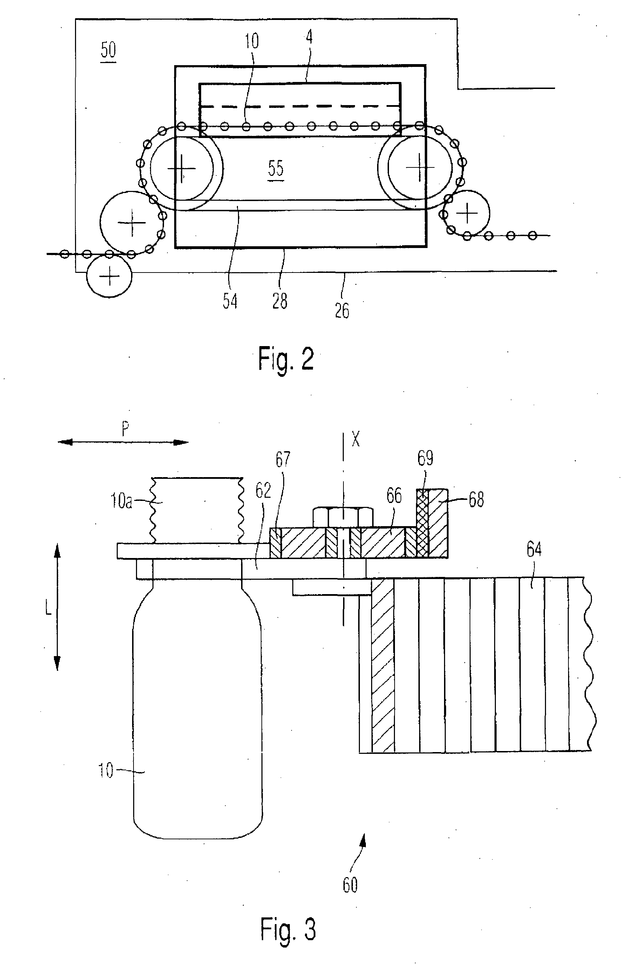

Fig. 2 zeigt eine teilweise Darstellung aus Fig. 1, genauer gesagt den Bereich der Vorrichtung 1, in dem die erste Sterilisationseinrichtung 4 angeordnet ist. Diese Sterilisationseinrichtung 4 kann dabei ebenfalls eine Transportkette 54 mit einer Vielzahl von Greifelementen aufweisen, welche die Behältnisse greifen. Bevorzugt sind diese Greifelemente derart gestaltet, dass sie eine Rotation der Vorformlinge 10 um deren eigene Längsachse erlauben, um auf diese Weise die Sterilisationswirkung zu verbessern.

Das Bezugszeichen 28 bezieht sich auf ein zweites Gehäuse, innerhalb dessen die Sterilisationseinrichtung 4 angeordnet ist. Dieses Gehäuse 28 ist wiederum vollständig innerhalb des oben erwähnten ersten Gehäuses 26 angeordnet. Innerhalb des zweiten Gehäuses 28 befindet sich ein zweiter Sterilraum 55, der insbesondere unter einem höheren Reinheitsgrad gehalten ist als der erste Sterilisationsraum 50.

Fig. 3 zeigt eine schematische Darstellung eines Greifelements 60 welches mit einer Greifklammer einen Vorformling 10 trägt. Dabei ist es möglich, dass dieses Greifelement eine Antriebseinrichtung 66 aufweist, welche eine Drehung des Vorformlings 10 um seine eigene Achse bewirkt. Das Bezugszeichen 64 bezieht sich auf einen Träger, der wiederum die Greifeinrichtung 62 hält. Es wäre jedoch auch möglich, dass mit Hilfe dieses Trägers 64 während des Transports der Vorformlinge noch weitere Bewegungen erzeugt werden wie beispielsweise in Fig. 3 eine Bewegung entlang des Doppelpfeils P. Umgekehrt wäre es jedoch auch möglich, dass die (nicht gezeigten) Strahlungseinrichtungen der ersten Sterilisationseinrichtung 4 sich beispielsweise in der Richtung P gegenüber den Vorformlingen 10 bewegen. Auch wäre es, wie oben gesagt, möglich, dass die entsprechenden Strahlungsquellen in einer Längsrichtung L des Vorformlings 10 oder auch der Richtung P bewegen.

Bei der Antriebseinrichtung 66 handelt es sich hier um ein Rad 66, welches an dem Greifelement 60 angeordnet ist und welches einerseits einen Tragring 10a des Vorformlings kontaktiert und andererseits einen stationären Rand 68, der beispielsweise in einem Bereich der ersten Sterilisationseinrichtung angeordnet sein kann. Bei einer Bewegung des Greifelements 60 in der Transporteinrichtung wird das Rad 66 relativ gegenüber dem Rand bewegt und dreht sich auf diese Weise. Dadurch wird auch der Vorformling über seinen Tragring oder allgemein denjenigen Bereich des Vorformlings, der von dem Rad 66 kontaktiert wird, gedreht.

Es wird darauf hingewiesen, dass das in Fig. 3 dargestellte Greifelement nicht nur zum Drehen von Vorformlingen 10, sondern auch zum Drehen der Behältnisse 20 verwendet werden kann. Vorzugsweise kontaktiert daher das Rad 66 einen Bereich der Mündung des Vorformlings bzw. Kunststoffbehältnisses 20, da dieser Bereich während des Blasvorgangs in der Regel unverändert bleibt. Das Rad 66 ist dabei um eine Achse X drehbar gelagert. Weiterhin kann das Rad 66 mit einem Gummiüberzug 67 oder dergleichen versehen sein, um die Reibung mit dem Tragring 10a des Behältnisses zu erhöhen. Auch der Rand 68 kann einen entsprechenden Gummiüberzug 69 aufweisen.

Die in Fig. 3 dargestellte Einrichtung erlaubt eine mechanisch sehr einfache Drehung der Behältnisse, wobei auch diejenigen Bereiche, in denen eine Drehung stattfindet, einfach bestimmt werden können, in dem entweder ein Rand 68 vorgesehen ist oder nicht. Auch wäre es möglich, den Rand 68 beweglich auszuführen, um ihn insbesondere von dem Rad wegbewegen zu können, um auf diese Weise die Drehung des Kunststoffbehältnisses ab- oder zuschalten zu können.

Die Greifeinrichtung bzw. Greifklammer 62 ist dabei derart ausgelegt, dass sie eine Drehung des Kunststoffvorformlings 10 oder Behältnisses 20 erlaubt, ohne dass dieses nach unten herausfallen kann. So kann beispielsweise die Greifklammer 62 an ihrer dem Kunststoffvorformling oder dem Kunststoffbehältnis zugewandten Oberfläche Lagerelemente wie Rollen aufweisen.

### Bezugszeichenliste

- 1: Vorrichtung
- 2: erste Transporteinrichtung
- 4: erste Sterilisationseinrichtung
- 5: Heizeinrichtung
- 6: Umformungseinrichtung
- 8: zweite Sterilisationseinrichtung
- 10: Kunststoffvorformling
- 10a: Mündung des Kunststoffvorformlings bzw. Kunststoffbehältnisses
- 11: Transportkette
- 12: zweite Transporteinrichtung
- 16: Blasrad
- 18: Transportrad
- 19: Transportrad
- 20: Kunststoffbehältnis
- 26: erstes Gehäuse
- 28: zweites Gehäuse
- 32: Transportkette
- 40: Befüllungseinrichtung
- 41: Transportrad
- 48: Transportrad
- 50: erster Sterilraum
- 55: zweiter Sterilraum
- 60: Greifelement
- 62: Greifklammer
- 64: Träger
- 66: Antriebseinrichtung, Rad
- 67: Gummiüberzug des Rads 66
- 68: Rand
- 69: Gummiüberzug des Rands 68

- L: Längsrichtung der Behältnisse
- P: Bewegungsrichtung

## Patentansprüche

1. Vorrichtung (1) zum Herstellen von Kunststoffbehältern (20) mit einer Umformungseinrichtung (6), welche Kunststoffvorformlinge (10) zu Kunststoffbehältnissen (20) umformt, mit einer ersten Transporteinrichtung (2), welche die Kunststoffvorformlinge (10) der Umformungseinrichtung (6) zuführt und einer zweiten Transporteinrichtung (12), welche die Kunststoffbehältnisse (20) von der Umformungseinrichtung (6) abführt,
**dadurch gekennzeichnet, dass**
die Vorrichtung eine erste Sterilisationseinrichtung (4) zum Sterilisieren der Kunststoffvorformlinge (10) aufweist, welche in einer Transportrichtung der Kunststoffvorformlinge (10) vor der Umformungseinrichtung (6) angeordnet ist und eine zweite Sterilisationseinrichtung (8) zum Sterilisieren der Kunststoffbehältnisse (20), welche in der Transportrichtung der Kunststoffbehältnisse (20) nach der Umformungseinrichtung (6) angeordnet ist und wobei die erste Sterilisationseinrichtung (4) eine Strahlungsquelle aufweist, welche Elektronenstrahlen auf die Kunststoffvorformlinge (10) richtet und die zweite Sterilisationseinrichtung eine Strahlungsquelle aufweist, welche die Kunststoffbehältnisse (20) mit UV -Strahlung beaufschlägt.

2. Vorrichtung (1) nach wenigstens einem der vorangegangenen Ansprüche,
**dadurch gekennzeichnet, dass**
die Vorrichtung (1) eine Dreheinrichtung aufweist, welche die Vorformlinge (10) während der Sterilisation durch die erste Sterilisationseinrichtung (4) dreht.

3. Vorrichtung (1) nach wenigstens einem der vorangegangenen Ansprüche,
**dadurch gekennzeichnet, dass**
wenigstens eine Sterilisationseinrichtung (4,8) wenigstens eine Strahlungsquelle (24) aufweist, deren Bewegung an die Bewegung der Kunststoffvorformlinge (10) oder die Bewegung der Kunststoffbehältnisse (20) gekoppelt ist.

4. Vorrichtung (1) nach wenigstens einem der vorangegangenen Ansprüche,
**dadurch gekennzeichnet, dass**
wenigstens ein Abschnitt wenigstens einer Strahlungsquelle in die Mündung (10a) der zu sterilisierenden Kunststoffvorformlinge (10) einführbar ist.

5. Vorrichtung (1) nach wenigstens einem der vorangegangenen Ansprüche,
**dadurch gekennzeichnet, dass**
die Vorrichtung eine Heizeinrichtung (5) aufweist, welche die Kunststoffvorformlinge (10) erwärmt.

6. Vorrichtung (1) nach Anspruch 5,
**dadurch gekennzeichnet, dass**
die erste Sterilisationseinrichtung (4) in der Transportrichtung der Kunststoffvorformlinge (10) vor der Heizeinrichtung (5) angeordnet ist.

7. Vorrichtung (1) nach wenigstens einem der vorangegangenen Ansprüche,
**dadurch gekennzeichnet, dass**
die Umformungseinrichtung (6), und die Sterilisationseinrichtungen (4, 8) innerhalb eines Gehäuses (26) angeordnet sind, wobei dieses Gehäuse (26) in seinem Inneren einen Sterilraum (50) ausbildet.

8. Vorrichtung (1) nach Anspruch 7,
**dadurch gekennzeichnet, dass**
im Bereich der ersten Sterilisationseinrichtung (4) innerhalb des Gehäuses (26) ein zweites Gehäuse (28) vorgesehen ist, wobei innerhalb dieses zweiten Gehäuses (28) die erste Sterilisationseinrichtung (4) angeordnet ist.

9. Verfahren zum Herstellen von Kunststoffbehältnissen (20) aus Kunststoffvorformlingen (10), wobei einer Umformungseinrichtung (6) Kunsstoffvorformlinge (10) mittels einer ersten Transporteinrichtung (2) zugeführt werden und aus den Kunststoffvorformlingen (10) erzeugte Kunststoffbehältnisse (20) mittels einer zweiten Transporteinrichtung (12) von der Umformungseinrichtung (6) abgeführt werden,
**dadurch gekennzeichnet, dass**
die Kunststoffvorformlinge (10) auf ihrem Transportpfad vor der Umformungseinrichtung (6) und die aus den Kunststoffvorformlingen (10) erzeugten Kunststoffbehältnisse (20) auf ihrem Transportpfad nach der Umformungseinrichtung (6) sterilisiert werden, wobei die erste Sterilisationseinrichtung (4) eine Strahlungsquelle aufweist, mittels derer Elektronenstrahlen auf die Kunststoffvorformlinge (10) gerichtet werden und die zweite Sterilisationseinrichtung (8) eine Strahlungsquelle aufweist, mittels derer die Kunststoffbehältnisse (20) mit UV -Strahlung beaufschlagt werden.

## Claims

1. An apparatus (1) for the production of plastics material containers (20) with a shaping device (6) which shapes plastics material pre-forms (10) into plastics material containers (20), with a first conveying device (2) which conveys the plastics material pre-forms (10) to the shaping device (6) and a second conveying device (12) which removes the plastics material containers (20) from the shaping device (6), **characterized in that** the apparatus has a first sterilization device (4) for sterilizing the plastics material pre-forms (10), which is arranged upstream of the shaping device (6) in a conveying direction of the plastics material pre-forms (10), and a second sterilization device (8) for sterilizing the plastics material containers (20), which is arranged downstream of the shaping device (6) in the conveying direction of the plastics material containers (20), and wherein the first sterilization device (4) has a radiation source which directs electron beams onto the plastics material pre-forms (10), and the second sterilization device has a radiation source which acts upon the plastics material containers (20) with UV radiation.

2. An apparatus (1) according to claim 1, **characterized in that** the apparatus (1) has a turning device which turns the pre-forms (10) during the sterilization by the first sterilization device (4).

3. An apparatus (1) according to at least one of the preceding claims, **characterized in that** at least one sterilization device (4, 8) has at least one radiation source (24), the movement of which is coupled to the movement of the plastics material pre-forms (10) or the movement of the plastics material containers (20).

4. An apparatus (1) according to at least one of the preceding claims, **characterized in that** at least one portion of at least one radiation source is capable of being introduced into the aperture (10a) of the plastics material pre-forms (10) to be sterilized.

5. An apparatus (1) according to at least one of the preceding claims, **characterized in that** the apparatus has a heating device (5) which heats the plastics material pre-forms (10).

6. An apparatus (1) according to claim 5, **characterized in that** the first sterilization device (4) is arranged upstream of the heating device (5) in the conveying direction of the plastics material pre-forms (10).

7. An apparatus (1) according to at least one of the preceding claims, **characterized in that** the shaping device (6) and the sterilization devices (4, 8) are arranged inside a housing (26), wherein this housing (26) forms a sterile room (50) in its interior.

8. An apparatus (1) according to claim 7, **characterized in that** a second housing (28) is provided inside the housing (26) in the region of the first sterilization device (4), wherein the first sterilization device (4) is arranged inside this second housing (28).

9. A method of producing plastics material containers (20) from plastics material pre-forms (10), wherein plastics material pre-forms (10) are supplied to a shaping device (6) by means of a first conveying device (2) and plastics material containers (20) produced from the plastics material pre-forms (10) are removed from the shaping device (6) by means of a second conveying device (12), **characterized in that** the plastics material pre-forms (10) are sterilized on their conveying path upstream of the shaping device (6) and the plastics material containers (20) produced from the plastics material pre-forms (10) are sterilized on their conveying path downstream of the shaping device (6), wherein the first sterilization device (4) has a radiation source by means of which electron beams are directed onto the plastics material pre-forms (10) and the second sterilization device (8) has a radiation source by means of which the plastics material containers (20) are acted upon with UV radiation.

## Revendications

1. Dispositif (1) pour la fabrication de récipients (20) en matière plastique, avec un dispositif de transformation (6) qui transforme des préformes (10) en matière plastique en récipients (20) en matière plastique, avec un premier dispositif de transport (2) qui amène les préformes (10) en matière plastique au dispositif de transformation (6) et un deuxième dispositif de transport (12) qui évacue les récipients (20) en matière plastique du dispositif de transformation (6),
**caractérisée en ce que**
ledit dispositif comporte un premier dispositif de stérilisation (4) pour stériliser les préformes (10) en matière plastique, lequel est situé en amont du dispositif de transformation (6) dans la direction de transport des préformes (10) en matière plastique, et un deuxième dispositif de stérilisation (8) pour stériliser les récipients (20) en matière plastique, lequel est situé en aval du dispositif de tranfsormation (6) dans la direction de transport des récipients (20) en matière plastique, et le premier dispositif de stérilisation (4) présentant une source de rayonnement qui dirige des rayons d'électrons vers les préformes (10) en matière plastique, et le deuxième dispositif de stérilisation présentant une source de rayonnement qui soumet les récipients (20) en matière plastique à un rayonnement UV.

2. Dispositif (1) selon la revendication précédente, **caractérisée en ce que**
ledit dispositif (1) présente un dispositif de rotation qui tourne les préformes (10) pendant la stérilisation par le premier dispositif de stérilisation (4).

3. Dispositif (1) selon au moins une des revendications précédentes, **caractérisée en ce qu'**
au moins un dispositif de stérilisation (4, 8) présente au moins une source de rayonnement (24) dont le déplacement est couplé au déplacement des préformes (10) en matière plastique ou au déplacement des récipients (20) en matière plastique.

4. Dispositif (1) selon au moins une des revendications précédentes, **caractérisée en ce qu'**
au moins une section d'au moins une source de rayonnement peut être introduite dans l'embouchure (10a) des préformes (10) en matière plastique à stériliser.

5. Dispositif (1) selon au moins une des revendications précédentes, **caractérisée en ce que**
ledit dispositif comporte un dispositif de chauffage (5) qui chauffe les préformes (10) en matière plastique.

6. Dispositif (1) selon la revendication 5, **caractérisée en ce que**
le premier dispositif de stérilisation (4) est situé en amont du dispositif de chauffage (5) dans la direction de transport des préformes (10) en matière plastique.

7. Dispositif (1) selon au moins une des revendications précédentes, **caractérisée en ce que**
le dispositif de transformation (6) et les dispositifs de stérilisation (4, 8) sont disposés à l'intérieur d'un carter (26), ledit carter (26) formant à son intérieur un compartiment stérile (50).

8. Installation (1) selon la revendication 7, **caractérisée en ce qu'**
un deuxième carter (28) est prévu à l'intérieur du carter (26) dans la région du premier dispositif de stérilisation (4), le premier dispositif de stérilisation (4) étant disposé à l'intérieur dudit deuxième carter (28).

9. Procédé de fabrication de récipients (20) en matière plastique à partir de préformes (10) en matière plastique, des préformes (10) en matière plastique étant amenées à un dispositif de transformation (6) au moyen d'un premier dispositif de transport (2) et les récipients (20) en matière plastique produits à partir des préformes (10) en matière plastique étant évacués du dispositif de transformation (6) au moyen d'un deuxième dispositif de transport (12),
**caractérisé en ce que**
les préformes (10) en matière plastique sont stérilisées en amont du dispositif de formage (6) sur leur chemin de transport et les récipients (20) en matière plastique produits à partir des préformes (10) en matière plastique en aval du dispositif de formage (6) sur leur chemin de transport, le premier dispositif de stérilisation (4) présentant une source de rayonnement au moyen de laquelle des rayons d'électrons sont dirigés vers les préformes (10) en matière plastique, et le deuxième dispositif de stérilisation (8) présentant une source de rayonnement au moyen de laquelle les récipients (20) en matière plastique sont soumis à un rayonnement UV.
